Europäisches Patentamt

European Patent Office

Office européen des brevets

(19)

(11) Publication number: **0 296 740**
**A2**

## EUROPEAN PATENT APPLICATION

(21) Application number: **88305308.4**

(22) Date of filing: **10.06.88**

(51) Int. Cl.⁴ **C08B 37/08 , A61K 47/00**

Claims for the following Contracting States: ES + GR.

(30) Priority: **11.06.87 GB 8713662**

(43) Date of publication of application:
**28.12.88 Bulletin 88/52**

(84) Designated Contracting States:
**AT BE CH DE ES FR GR IT LI LU NL SE**

(71) Applicant: **SKANDIGEN AB**
**Norrlandsgatan 15**
**S-111 43 Stockholm(SE)**

(72) Inventor: **Ellwood, Derek Clifford c/o**
**Fermentech Limited**
**Research Avenue Two Heriot-Watt University**
**Campus**
**Riccarton Currie Edinburgh EH14 4AS(GB)**

(74) Representative: **Collier, Jeremy Austin Grey et al**
**J.A.Kemp & Co. 14, South Square Gray's Inn**
**London WC1R 5EU(GB)**

(54) Hyaluronic acid derivatives and their production.

(57) Conjugates of hyaluronic acid with a physiologically active or chemotherapeutic substance, e.g. an antibiotic, covalently bound thereto, have valuable delayed release properties. They may be made by the coupling of hyaluronic acid with the said substance in the presence of a suitable coupling agent, e.g. cyanogen bromide.

EP 0 296 740 A2

# HYALURONIC ACID DERIVATIVES AND THEIR PRODUCTION

This invention relates to hyaluronic acid derivatives and their production.

Hyaluronic acid is a linear polymer of N-acetyl glucosamine and glucuronic acid units. It is widely distributed in nature and is found in many mammalian species. It is also produced by some bacteria, e.g. strains of Streptococci such as S.equi. The molecule forms a tight coil which intertwines on itself. Aqueous solutions of hyaluronic acid are highly viscous. It is known to be immunologically inert. It has been proposed to use hyaluronic acid in therapy, e.g. to promote wound healing, and it has also been proposed for use in cosmetics.

It has now been found that conjugates of hyaluronic acid with physiologically active or chemotherapeutic substances are valuable therapeutic agents where slow release of the substance is required.

The present invention accordingly provides conjugates of hyaluronic acid (which may be in the form of the free acid or of a salt) with a physiologically active or chemotherapeutic substance covalently bound thereto. Such conjugates may be made, in accordance with a feature of the invention, by reacting hyaluronic acid (as the free acid or a salt) and the said physiologically active or chemotherapeutic substance with a coupling agent which is capable of reacting with one or more hydroxyls or carboxyls present in the hyaluronic acid and one or more functional groups present in the said substance.

Preferably, the physiologically or chemotherapeutic substance contains free primary amino groups through which the coupling to the hyaluronic acid can take place. It is especially advantageous to couple amino-group-containing antibiotic substances, e.g. penicillins, to hyaluronic acid.

The conjugates produced in accordance with the present invention have in general the same physiological activity and/or chemotherapeutic utility as the parent substance from which they are made. However, the coupling to the hyaluronic acid delays the release of the physiologically active or chemotherapeutic substance in vivo and in vitro and may also assist the penetration of the substance to the physiological site where it is required, e.g. by assisting penetration through the skin.

The proportion of physiologically active or chemotherapeutic substance which is coupled to the hyaluronic acid may vary within wide limits. Normally, the conjugate contains from 1 to 50% preferably 1 to 10% by weight of the physiologically active or chemotherapeutic substance. The optimum proportion in any particular context can readily be found by simple experiment.

To make the novel conjugates, the hyaluronic acid may be dissolved in a suitable solvent, e.g. water, and activated by reaction with an appropriate coupling reagent of a kind known for use in bringing about covalent bonding between functional groups. For example, cyanogen bromide may be used to couple the hyaluronic acid via its hydroxyl groups to amino-group-containing substances. Similar results may be obtained with reagents such as water soluble carbodiimides, N-ethoxycarbonyl-2-ethoxy-1,2 dihydroquinoline, and 2-ethyl-5-phenyl-isoxuzolium-3'-sulfonate (Woodwards Reagent K) which couple the hyaluronic acid via its carboxyl groups. Other coupling agents may, of course, be used, the choice being determined by the nature of the reactive functional groups which are present in the physiologically active or chemotherapeutic substance.

For the coupling of amino group containing antibiotics, it is preferred to use cyanogen bromide as the coupling agent. The hyaluronic acid, dissolved in water, is reacted in an alkaline medium with the cyanogen bromide. When the reaction is complete, the pH of the mixture is adjusted to near neutrality with hydrochloric acid, and the amino group containing antibiotic or other physiologically active or chemotherapeutic substance is then added. When the coupling reaction is complete, the conjugate of the hyaluronic acid and the antibiotic may be precipitated by pouring the reaction mixture into absolute ethanol or another water-miscible organic solvent which is a non-solvent for the conjugate.

The reaction may be effected at any temperature which does not adversely affect the hyaluronic acid or the antibiotic. Temperatures in the range of 0 to 50°C, and preferably ambient temperature, may be used. The proportion of cyanogen bromide may be, for example, from 0.5 to 2 parts by weight for each part by weight of the hyaluronic acid. The proportion of the antibiotic is preferably 5 to 20 parts by weight for each 100 parts by weight of the hyaluronic acid.

The following Example illustrates the invention.

## EXAMPLE

Hyaluronic acid (100 mg; commercially available material obtained by fermentation of Streptococcus equi, molecular weight $1.2 \times 10^6$, from Fermentech Ltd.) was dissolved in water (50 ml) with magnetic stirring under a nitrogen atmosphere.

The thick clear solution obtained was adjusted to pH 11 by addition of saturated sodium carbonate solution. Cyanogen Bromide (100 mg) was then added and the mixture was stirred for one hour under nitrogen, the pH being maintained at about 10.5 by dropwise addition of sodium hydroxide solution (2M). The pH was then adjusted to 8 by addition of hydrochloric acid (0.5M) and Ampicillin (12mg) was then added to the reaction mixutre. The latter was stirred under nitrogen for a further two hours and the reaction mixture was then poured into twice its volume of absolute ethanol and the mixture was kept at 4°C overnight. The stringy precipitate was filtered off, washed several times with absolute ethanol, and dried in air. It then weighed 94mg.

Samples of the conjugate obtained in this manner were dissolved in water to give a viscous solution. This solution was "spotted" onto a lawn plate of Staphylococcus aureus. After incubation overnight at 37°C, a small clear zone of inhibition of bacterial growth was observed around each drop of the conjugate. After further incubation at 37°C, the zones of inhibition increased. This shows that the ampicillin in the conjugate is still active against Staphylococcus aureus. Probably, the free antibiotic is# slowly released from the conjugate by the action of enzymes released by the microorganism.

Conjugates of hyaluronic acid with appropriate amino-group-containing antibiotics such as ampicillin can be used for topical application to the skin, e.g. in the treatment of acne vulgaris. For such a purpose, the novel conjugate is incorporated in a conventional medium for topical application to the skin which is compatible with the conjugate (i.e. does not inactivate the ampicillin), and the composition, containing, for example, 1 to 5% by weight of the conjugate, is then applied topically to the skin where treatment is required in the same way as known anti-bacterial compositions for topical application.

**Claims**

1. A conjugate of hyaluronic acid (in the form of a free acid or a salt) with a physiologically active or chemotherapeutic substance covalently bound thereto.

2. A hyaluronic acid conjugate according to claim 1 in which the physiologically active or chemotherapeutic substance contains a free primary amino group through which it is covalently bound to the hyaluronic acid.

3. A hyaluronic acid conjugate according to claim 2 in which the physiologically active or chemotherapeutic substance is an antibiotic which contains an amino group.

4. A hyaluronic acid conjugate according to claim 3 in which the said antibiotic is a penicillin.

5. A hyaluronic acid conjugate according to any one of claims 1 to 4 in which the physiologically active or chemotherapeutic substance makes up from 1 to 50% of the weight of the conjugate.

6. Process for the preparation of a hyaluronic acid conjugate as claimed in any one of claims 1 to 5 which comprises reacting hyaluronic acid (as the free acid or the salt) and the said physiologically active or chemotherapeutic substance with a coupling agent which is capable of reacting with one or more hydroxyls or carboxyls present in the hyaluronic acid and one or more functional groups present in the said substance.

7. Process according to claim 6 in which the said coupling agent is cyanogen bromide, a water soluble carbodiimide, N-ethoxycarbonyl-2-ethoxy-1,2-dihydroquinoline, or 2-ethyl-5-phenyl-isoxuzolium-3'-sulfonate.

8. Process according to claim 6 in which the hyaluronic acid is reacted in an aqueous alkaline medium with cyanogen bromide, the pH of the reaction mixture is adjusted to near neutrality with an acid, and a physiologically active or chemotherapeutic substance containing a primary amino group is then added.

9. Process according to claim 8 in which the said substance is an antibiotic containing an amino group.

10. Process according to claim 8 or 9 in which 0.5 to 2 parts by weight of cyanogen bromide are used for each part by weight of hyaluronic acid, and 5 to 20 parts by weight of the amino-group-containing substance are then added for each 100 parts by weight of the hyaluronic acid initially used.

Claims for the following contracting States: GR, ES

1. Process for the preparation of a conjugate of hyaluronic acid (in the form of a free acid or a salt) with a physiologically active or chemotherapeutic substance covalently bound thereto, which comprises reacting hyaluronic acid (as the free acid or the salt) and the said physiologically active or chemotherapeutic substance with a coupling agent which is capable of reacting with one or more hydroxyls or carboxyls present in the hyaluronic acid and one or more functional groups present in the said substance.

2. Process according to claim 1 in which the said coupling agent is cyanogen bromide, a water soluble carbodiimide, N-ethoxycarbonyl-2-ethoxy-1,2-dihydroquinoline, or 2-ethyl-5-phenyl-isoxuzolium-3'-sulfonate.

3. Process according to claim 1 in which the hyaluronic acid is reacted in an aqueous alkaline medium with cyanogen bromide, the pH of the reaction mixture is adjusted to near neutrality with an acid, and a physiologically active or chemotherapeutic substance containing a primary amino group is then added.

4. Process according to claim 3 in which the said substance is an antibiotic containing an amino group.

5. Process according to claim 4 in which the said antibiotic is a penicillin.

6. Process according to claim 3, 4 or 5 in which 0.5 to 2 parts by weight of cyanogen bromide are used for each part by weight of hyaluronic acid, and 5 to 20 parts by weight of the amino-group-containing substance are then added for each 100 parts by weight of the hyaluronic acid initially used.

7. Process according to any one of claims 1 to 6 in which the physiologically active or chemotherapeutic substance makes up from 1 to 50% of the weight of the conjugate.